# EUROPEAN PATENT APPLICATION

(11) **EP 1 612 559 A1**
(43) Date of publication of application: **04.01.2006**
(21) Application number: 04722400.1
(22) Date of filing: 22.03.2004
(51) Int. Cl.: G01N 33/92

(54) **METHOD FOR EVALUATING ANTIOXIDANT POTENTIAL OF BIOLOGICAL SAMPLE**

(30) Priority: 20.03.2003 JP 2003077382
(71) Applicant: CHUGAI SEIYAKU KABUSHIKI KAISHA, Tokyo, 115-8543 (JP); THE HEART RESEARCH INSTITUTE LIMITED, Camperdown, NSW 2050 (AU)
(72) Inventor: CYNSHI, Osamu, c/o Chugai Seiyaku Kabushiki Kaisha, Gotenba-shi, Shizuoka 4128513 (JP); STOCKER, Roland, Cronulla, New South Wales 2230 (AU)
(74) Representative: Vossius & Partner
(86) International application number: PCT/JP2004/003873
(87) International publication number: WO 2004/083869

(57) **Abstract**

Herein provided is a method for evaluating the antioxidant potential of a biological sample comprising the steps of:
(a) collecting a sample containing at least oxidizable substrates from a living body;
(b) initiating an oxidation reaction of said oxidizable substrates;
(c) continuing said oxidation reaction; and
(d) quantifying oxidation products formed from said oxidizable substrates by determining the rate of said oxidation reaction during the progress thereof or by performing an assay after said oxidation reaction has been stopped,

wherein said steps (b) and (c) are performed in the presence of one or more antioxidant components.

## Description

### TECHNICAL FIELD

The present invention relates to a method for evaluating in vivo antioxidant potential against oxidative stress.

### BACKGROUND ART

Aerobic organisms including humans gain energy via oxidative phosphorylation with oxygen. This oxidative phosphorylation proceeds in the presence of oxygen radicals generated from oxygen. Thus, the presence of oxygen radicals is essential for living bodies.

However, oxygen radicals are also thought to cause oxidative stress by oxidizing in vivo tissues or biological components and known to exacerbate various diseases such as cataract, diabetes, Alzheimer's disease, cancers, arteriosclerosis, cardiopulmonary diseases, chronic inflammatory diseases and ischemic diseases (Sies H. (1977) Oxidative stress: oxidants and antioxidants, Review. Exp. Physiol. 82: 291-5; Scott G. (1997) Antioxidants in science, technology, medicine and nutrition. Coll House, UK Publishing; Porter N. A. (1990) Auto-oxidation of polyunsaturated fatty acids: Initiation, propagation and product distribution (basic chemistry). Vigo-Pelfrey, C. ed Membrane lipid oxidation. Vol. 1 Boca Raton, FL., CRC Press; Simic M., Karel M. (1980) Auto oxidation in food and biological systems. New York, Plenum Press). Living bodies are always exposed to oxidative stress caused by oxygen radicals due to the constant in vivo energy production mediated by oxygen radicals.

Living bodies have antioxidant potential as means for protecting individuals against this oxidative stress. The level of this antioxidant potential varies with individuals so that some individuals are resistant to the diseases as described above even under considerably heavy oxidative stress while others are susceptible.

Therefore, if the antioxidant potential of individuals against the above diseases to be exacerbated by oxidative stress could be exactly evaluated, useful information could be obtained for preventing or treating such diseases.

Known methods for evaluating antioxidant potential rely on assaying oxidation products already oxidized in vivo or collecting an unoxidized biological sample and inducing the oxidization of the sample ex vivo to assess the resistance of the sample to oxidation.

For assaying oxidation products already oxidized in vivo, lipoperoxides which are in vivo oxidation products and are TBA-reactive have been assayed (Yagi K. A simple fluorometric assay for lipoperoxide in blood plasma. Biochem Med. 1976 15:212-6). In this assay, the amount of lipoperoxides is determined by measuring the intensity of fluorescence which is emitted by heating the lipoperoxides with TBA under an acidic condition. However, this assay does not give any information about the site where the lipoperoxides were produced in vivo and the reaction through which they were produced, so that one cannot know what kind of tissue and what kind of oxidation reaction are covered by the antioxidant potential determined from the measured values. Moreover, such an assay of oxidation products already oxidized in vivo is insufficient for evaluating in vivo antioxidant potential because lipoperoxides are also produced by in vivo enzymatic reactions and their concentrations are inevitably influenced by metabolism or excretion.

It has been reported that a recently developed assay for oxidation products in which isoprostane F2α is subjected to measurement avoids the influence of enzymatic oxidation reactions and thus allows one to assay only oxidation products produced by radical reactions, indicating that this assay allows one to evaluate an antioxidant potential reflecting only such oxidation reactions (Patrono C, FitzGerald GA. Isoprostanes: potential markers of oxidant stress in atherothrombotic disease. Arterioscler Thromb Vasc Biol. 1997 17:2309-15). However, one cannot know what kind of tissue and what kind of radical oxidation reaction are covered by the antioxidant potential, and the influence of the metabolism or excretion of the oxidation products is still inevitable.

On the other hand, in an assay in which ex vivo oxidation of a biological sample is induced, a sample is collected from a living body and subjected to an ex vivo oxidation reaction to assess the antioxidant potential of in vivo antioxidant components contained in the sample, whereby the antioxidant potential in a specific tissue from which the biological sample was collected can be evaluated.

In this assay, a TAS assay using a sample such as, for example, a serum or tissue homogenate has been used for a clinical test (Total Antioxidant Status: Rice-Evans C, Miller NJ. Total antioxidant status in plasma and body fluids. Methods Enzymol. 1994 234:279-93). In the TAS assay, an oxidation reaction in the sample is induced by radicals generated from iron ions in the assistance of oxidizers, hydrogen peroxide and metmyoglobin. Measurement of the ability of the sample to resist this oxidation reaction allows one to evaluate the antioxidant potential of the sample. This assay can be performed with autoanalyzers because of the short reaction time of typically 6 minutes. An assay called TRAP (Total Radical-trapping Antioxidant Potential) has also been developed wherein the combination of hydrogen peroxide and metmyoglobin was replaced by 2,2'-azobis (2-amidinopropane) hydrochloride (AAPH) which is capable of constantly generating radicals (Wayner DD, Burton GW, Ingold KU, Locke S. Quantitative measurement of the total, peroxyl radical-trapping antioxidant capability of human blood plasma by controlled peroxidation. The important contribution made by plasma proteins. FEBS Lett. 1985 187:33-7). In these assays, large amounts of radicals generated in a short period consume antioxidant materials present in samples, suggesting that direct reactions may occur between the radicals generated for initiating oxidation and the antioxidant materials. As a result, the total amount of antioxidant components capable of participating in oxidative reactions with radicals is regarded as contributing to the antioxidant potential of the sample.

Thus, the assay relying on inducing ex vivo oxidation of a biological sample allows one to evaluate the antioxidant potential of a specific component capable of reacting with radicals in the sample.

Assays using LDL samples are also available, including Esterbauer's assay proposed by Esterbauer (Esterbauer H, Striegl G, Puhl H, Rotherneder M. Continuous monitoring of in vitro oxidation of human low density lipoprotein. Free Radic Res Commun. 1989 6:67-75). In this Esterbauer's assay, a lipid peroxidation chain reaction is induced ex vivo by heating an LDL sample with copper ions at 37°C to evaluate the antioxidant potential of the LDL sample against the lipid peroxidation reaction. This assay assesses the production of an oxide by the absorbance at 234 nm, and assumes that the time lag from the start of the reaction to the initial detection of changes in the absorbance at 234 nm reflects the amount of antioxidant components in a sample. A report shows that administration of α-tocopherol induces prolongation of the time lag in humans in this assay (Dieber-Rotheneder M, Puhl H, Waeg G, Striegl G, Esterbauer H. Effect of oral supplementation with D-alpha-tocopherol on the vitamin E content of human low density lipoproteins and resistance to oxidation. J Lipid Res. 1991 32:1325-32).

In all of the TAS, TRAP and Esterbauer's assays described above, an oxidation reaction is carried out ex vivo until antioxidant components contained in a sample are exhausted, and the total amount of the antioxidant components thus exhausted is regarded as corresponding to the antioxidant potential of the sample. A major antioxidant component in the sample used in these evaluation methods is thought to be α-tocopherol, which is a molecular species having the highest antioxidant activity among vitamins E (Antioxidant Components, Free Radicals and Biological Protection, edited by Niki, Shimazaki and Mino, published by Gakkai Shuppan Center, 1994). Thus, it can be said that, in these evaluation methods, the total amount of α-tocopherol in a sample is regarded as corresponding to antioxidant potential of the sample. As a result, if a sample shows a high α-tocopherol level, the individual from which the sample is collected will be evaluated to have high antioxidant potential, i.e., to be resistant to diseases to be exacerbated by oxidative stress, such as arteriosclerosis and ischemic diseases. In fact, kits for conducting these assays are commercially available from Randox Laboratories Ltd., OXIS International, Inc., etc., as useful tools for clinical diagnoses.

However, a report shows that α-tocopherol is not exhausted and remains even in arteriosclerotic lesions with advanced oxidation in vivo (Upston JM, Terentis AC, Morris K, Keaney Jr JF, Stocker R. Oxidized lipid accumulates in the presence of alpha-tocopherol in atherosclerosis. Biochem J. 2002 1; 363:753-60). This suggests that, even in the presence of in vivo antioxidant components of which α-tocopherol is representative, an oxidation proceeds in lesions, such as arteriosclerotic lesions, to be exacerbated by oxidative stress. In other words, this suggests that there occurs an oxidation which cannot be inhibited by such in vivo antioxidant components.

A recent report shows that α-tocopherol promotes lipid peroxidation under specific experimental conditions (Maiorino M, Zamburlini A, Roveri A, Ursini F. Prooxidant role of vitamin E in copper induced lipid peroxidation. FEBS Lett. 1993 330:174-6). A method for determining the lipid peroxidation-promoting effect of α-tocopherol was also reported (Witting PK, Mohr D, Stocker R. Assessment of prooxidant activity of vitamin E in human low-density lipoprotein and plasma. Methods Enzymol. 1999;299:362-75). This phenomenon is considered to be a lipid peroxidation-promoting effect specific to α-tocopherol (Upston JM, Terentis AC, Stocker R. Tocopherol-mediated peroxidation of lipoproteins: implications for vitamin E as a potential antiatherogenic supplement. FASEB J. 1999 13:977-94), and such effect is considered to be one of reasons why oxidation proceeds even in the presence of the antioxidant component.

This indicates that the true risk of lesions to be exacerbated by oxidative stress has not been shown by conventional evaluation methods of antioxidant potential. Karmansky et al. reported that a good correlation was not always shown between the level of the evaluated antioxidant potential and the severity of condition (Karmansky I, Shnaider H, Palant A, Gruener N. Plasma lipid oxidation and susceptibility of low-density lipoproteins to oxidation in male patients with stable coronary artery disease. Clin Biochem. 1996 29:573-9).

Thus, if the products of oxidation reactions which proceed even in the presence of in vivo antioxidant components are assayed, the risk of lesions to be exacerbated by oxidative stress will be more exactly evaluated.

As for identification of products of oxidative reactions of biological samples for the purpose of defining the stages of LDL oxidation, a method for separating cholesterol ester hydroperoxides (CEOOHs) and cholesterol ester hydroxides (CEOHs), which are oxidation products of cholesterol esters, using HPLC has been established (L. Kritharides, W. Jessup, J. Gifford, and R. T. Dean, A method for defining the stages of low-density lipoprotein oxidation by the separation of cholesterol- and cholesterol ester-oxidation products using HPLC. Analytical Biochemistry 213, 79-89 (1993)).

### DISCLOSURE OF THE INVENTION

The present invention provides a method for evaluating in vivo antioxidant potential against oxidation occurring in the presence of antioxidant components.

As a result of studies focused on the presence of an oxidation reaction which proceeds even in the presence of antioxidant components, we found a method for assaying such oxidation products.

The present invention relates to a method for evaluating the antioxidant potential of a biological sample comprising the steps of:
(a) collecting a sample containing at least oxidizable substrates from a living body;
(b) initiating an oxidation reaction of said oxidizable substrates;
(c) continuing said oxidation reaction; and
(d) quantifying oxidation products formed from said oxidizable substrates by determining the rate of said oxidation reaction during the progress thereof or by performing an assay after said oxidation reaction has been stopped,

wherein said steps (b) and (c) are performed in the presence of one or more antioxidant components.

The present invention also relates to a method for diagnosing a disease or evaluating the prognosis and/or predicting the progress of said disease of a patient of interest on the basis of the antioxidant potential of a biological sample evaluated by the method described above.

The present invention also relates to a method for evaluating the antioxidant potential of an antioxidant test component comprising the steps of:
(a) collecting a sample containing at least oxidizable substrates from a living body;
(b) adding said antioxidant test component to said biological sample;
(c) initiating an oxidation reaction of the mixture of step (b);
(d) continuing said oxidation reaction; and
(e) quantifying oxidation products formed from said oxidizable substrates by determining the rate of said oxidation reaction during the progress thereof or by performing an assay after said oxidation reaction has been stopped,

wherein said steps (c) and (d) are performed in the presence of one or more antioxidant components.

### BRIEF DESCRIPTION OF THE DRAWING

FIG. 1 is a representative HPLC chart of the oxidation reaction solution of Example 1 after the oxidation reaction has been stopped. The peak at a retention time of about 9 minutes is attributed to CEOOH and CEOH, and the peak at a retention time of about 5 minutes is attributed to α-tocopherol.
FIG. 2 is a graph showing the relationship between AAPH concentrations and the amount of oxidation products. The abscissa represents AAPH concentration and the ordinate represents the total amount of cholesterol ester hydroperoxides (CEOOHs) and cholesterol ester hydroxides (CEOHs) produced.
FIG. 3 is a representative HPLC chart of the oxidation reaction solution of Example 2 in which α-tocopherol has exhausted.
FIG. 4 is a graph showing changes in the amount of CEOOHs and CEOHs produced in the presence of α-tocopherol, probucol or BO-653 at varying concentrations.

### DETAILED DESCRIPTION OF THE INVENTION

In an aspect, the present invention provides a method for evaluating the antioxidant potential of a biological sample comprising initiating an oxidation reaction ex vivo in a biological sample containing antioxidant components and oxidizable substrates, continuing the oxidation reaction in a condition where the antioxidant components are not totally exhausted by oxidizing species, and quantifying the oxidized products formed from the oxidizable substrates by this oxidation reaction. By this method, the antioxidant potential of the biological sample, accordingly, the antioxidant potential of the individual from which the biological sample is derived, is evaluated.

Target living bodies according to this method are mammals, preferably humans. Biological samples include tissue homogenates, lymph fluid, urine, blood, plasma and serum. Tissues from which samples are collected include, but not limited to, skin, liver, vascular wall, blood and urine, preferably blood. The samples may be used at their in vivo concentrations or diluted up to 10000:1, preferably up to 10:1. It is preferred to dilute with physiological saline. Optionally, a chelator may be added to protect the samples against oxidation. Generally, the pH is preferably adjusted to 3-10.

The term "antioxidant components" which are inherently contained in a biological sample in the method of the present invention collectively refers to easily oxidizable materials possessed by living bodies to resist oxidative stress, especially α-tocopherol. The antioxidant components have been thought to compensate for oxidizing species by being oxidized ahead of oxidizable substrates coexisting in the sample, thereby acting to prevent the oxidizable substrates from being oxidized. However, they do not seem to always prevent oxidizable substrates from being oxidized because α-tocopherol was reported to promote lipid peroxidation under specific experimental conditions as described above.

The term "oxidizable substrates" used in the method of the present invention means, for example, lipids such as cholesterol esters, neutral lipids or phospholipids; hemoglobin; or proteins having an SH group when the sample is blood, plasma or serum, among which cholesterol esters are major oxidizable substrates.

The oxidation reaction in the method of the present invention can be initiated by adding an oxidation initiator or by exposing the sample to UV rays or radiations or by autooxidation. However, the use of an oxidation initiator is a preferred initiation means because the intensity of the oxidation reaction can be readily controlled by selecting the type or the amount of the initiator to be added. Suitable oxidation initiators include, for example, peroxides such as hydrogen peroxide and t-butyl hydroperoxide; metal salts such as iron chloride and copper sulfate; azo initiators such as 2,2'-azobis (2-amidinopropane) hydrochloride (AAPH), 2,2'-azobis (2,4-dimethylvaleronitrile) and 2,2'-azobis (4-methoxy-2,4-dimethylvaleronitrile); transition metals such as copper, iron and hemin; hypochlorous acid; heme iron, and hemoglobin. Enzymes may also be used as oxidation initiators, including, for example, lipoxidases, cyclooxygenases and lactoperoxidases.

These oxidation initiators may be used alone or as a combination of two or more of them. The use of AAPH alone is preferred for the ability of AAPH to control the oxidation reaction at an appropriate intensity.

In a specific embodiment of the present invention, AAPH is used as an oxidation initiator, the biological sample is plasma, and the AAPH concentration is 5-30 mM, preferably 5-20 mM, more preferably 8-12 mM, most preferably 10 mM. When the oxidation reaction is initiated by irradiation, UV rays or radiations are preferably used. Autooxidation can be initiated by exposing the sample to air or oxygen. The temperature at which oxidation is initiated is normally 20-50°C, preferably 37°C.

As used herein, the expression "in the presence of one or more antioxidant components" means a condition in which the antioxidant components have not been totally exhousted by redox reactions with oxidizing species. By maintaining such a condition, a circumstance under which such an oxidation that is unpreventable or rather promoted by antioxidant components which had been thought to inhibit the oxidation reaction of oxidizable substrates proceeds is created. Optionally, a condition in which antioxidant components are present may be artificially created by adding at least one antioxidant materials to the biological sample. In this case, the antioxidant materials to be added may be identical with or different from the antioxidant components inherently contained in the biological sample, but they are preferably identical with antioxidant components inherently contained in the biological sample. More preferably, only one antioxidant material is used and it is identical with one of the antioxidant components. Antioxidant materials that are different from antioxidant components inherently contained in the biological sample include fat-soluble antioxidant compounds such as butylated hydroxytoluene (BHT) and watersoluble antioxidant compounds such as Trolox.

The type of the oxidation reaction to be initiated and then continued depends on the initiating means. Generally, when the oxidation is initiated by an oxidation initiator or irradiation, it proceeds radically. Specific conditions for the oxidation reaction may be those under which the oxidation of oxidizable substrates proceeds and the antioxidant components remain even after the oxidation reaction stops. However, various specific conditions for the oxidation reaction where the concentration of the oxidation initiator, temperature, period and the like are varied can be employed. The reaction temperature is 20-50°C, preferably 37°C. Generally, the oxidation reaction is carried out at the temperature at which it is initiated. The period for which the oxidation reaction is continued is preferably 2-10 hours, more preferably 4-8 hours.

In a specific embodiment of the present invention, the biological sample is plasma, the oxidation initiator is AAPH at a concentration of 5-30 mM, preferably 5-20 mM, more preferably 8-12 mM, most preferably 10 mM, the reaction temperature is 20-50°C, preferably 37°C, and the reaction period is within 24 hours, preferably within 8 hours.

Oxidation products formed from oxidizable substrates can be quantified by determining the rate of the oxidation reaction or by determining the amounts of the components contained in the oxidation reaction solution after the oxidation reaction has been stopped.

Means for determining the rate of the oxidation reaction include measuring the rate at which oxidizable substrates decrease by oxidation, or monitoring the progress of oxidation on the basis of the variation of the amount of a marker substance added, or measuring the rate at which the oxidation products generated by oxidation of oxidizable substrates increase. Specific methods for measuring the rate at which oxidizable substrates decrease by oxidation include, for example, measuring the amount of oxygen consumed, measuring the decrease of unsaturated lipids or measuring the decrease of glutathione. Specific methods for monitoring the progress of oxidation on the basis of the variation of the amount of a marker substance added include, for example, using a spin trapping agent or a fluorescent material as a marker substance. Methods for measuring the rate at which the oxidation products generated by oxidation of oxidizable substrates increase are similar to those as described below for quantification of the oxidized products formed from oxidizable substrates in the oxidation reaction solution after the oxidation reaction has been stopped.

When the oxidation products are quantified by determining the amounts of the components contained in the oxidation reaction solution after the oxidation reaction has been stopped, the quantification may be achieved by measuring the amount of oxidized oxidizable substrates or by determining the oxidation rate from the ratio between the concentration of oxidized oxidizable substrates and the concentration of unoxidized substrates. In either case, the reaction is normally stopped or attenuated by quenching such as immersing the vessel containing the oxidation reaction solution in ice water or inhibiting oxidation by adding a potent antioxidant. Then, oxidized and/or unoxidized oxidizable substrates are separated from the oxidation reaction solution. Separation is accomplished by extraction, electrophoresis, column chromatography or the like. Extraction with an organic solvent is preferred. Suitable organic solvents for this purpose include n-hexane, chloroform, methanol, ethanol, butanol, acetone, acetic acid, water and mixed solutions thereof. Mixed solutions of methanol/n-hexane containing acetic acid are preferred. The amount of the organic solvent used for extraction is 0.1-1000 times, preferably 1-100 times that of the biological sample or a dilution thereof. Separation may be omitted in the case of highly specific assays such as assays using antibodies.

The marker substance added to monitor the progress of oxidation and the means for quantifying oxidized or unoxidized oxidizable substrates depend on the types of the oxidizable substrates. When the analytes are lipids such as cholesterol esters, neutral lipids and phospholipids or oxidation products thereof, conventional assays for oxidized lipids or TBA reactive materials can be employed. When the analytes are proteins such as hemoglobin or oxidation products thereof, conventional assays for proteins or oxidatively denatured proteins can be employed. HPLC, LC-EDC, TLC, mass spectrometry, ELISA, a chromogenic assay based on changes in absorbance or an assay based on changes in fluorescence intensity can also be employed. However, HPLC or ELISA is preferred because it makes it possible not only to assay any type of analytes but also to identify the target analytes. HPLC equipped with a UV detector is especially preferred in terms of operability and costs, etc.

In one embodiment of the present invention wherein the oxidizable substrates are lipids, the oxidation products formed from the lipids include lipid peroxides having peroxy bond(s) (-O-O-), lipid oxides having oxy bond(s) (-O-), and lipids having diene bond (s) formed by elimination of the peroxy and/or oxy bond(s). Therefore, when the oxidizable substrates are cholesterol esters, the resulting oxidation products include, for example, cholesterol ester hydroperoxides, cholesterol ester hydroxides, sterol ester oxides and cholesterol ester aldehydes, among which the total amount of cholesterol ester hydroperoxides and cholesterol ester hydroxides are preferably determined.

When the oxidizable substrates are cholesterol esters, the in vivo antioxidant component is α-tocopherol, and oxidation reaction is initiated with AAPH and then continued for 8 hours, the concentration of AAPH should be preferably 8-12 mM, most preferably 10 mM, in order to maintain a condition where an antioxidant component is present during the oxidation reaction.

According to another aspect of the present invention, one can diagnose a disease or evaluate the prognosis and/or predict the progress of said disease of a patient of interest on the basis of the antioxidant potential of a biological sample evaluated by the method described above. Target diseases and/or conditions include diseases for which oxidative stress is known to be an exacerbating factor, such as cataract, diabetes, Alzheimer's disease, cancers, arteriosclerosis, cardiopulmonary diseases, hypercholesterolemia, chronic inflammatory diseases and ischemic diseases, especially diseases for which oxidative stress is high responsible, for example, chronic endocrine diseases such as diabetes; brain diseases such as Alzheimer's disease; pulmonary diseases such as chronic obstructive pulmonary disease (COPD); hepatic diseases such as nonalcoholic steatohepatitis (NASH); renal diseases such as chronic renal failure; circulatory diseases such as arteriosclerosis; chronic inflammatory diseases such as Crohn's disease and rheumatism; and cancers. This evaluation can also be used as an indicator for medical examinations.

Another aspect of the present invention provides a method for evaluating the antioxidant potential of an antioxidant test component comprising adding the antioxidant test component to a biological sample containing antioxidant components and oxidizable substrates, initiating an oxidation reaction, continuing the oxidation reaction in a condition where the antioxidant components are not totally exhausted by oxidizing species, and quantifying the oxidized products formed from the oxidizable substrates by this oxidation reaction.

This method intends to evaluate the antioxidant potential of an antioxidant test component by placing the test component under the mechanism where an oxidation reaction of oxidizable substrates contained in a biological sample occurs in the presence of antioxidant components. The evaluation target is the antioxidant potential of the antioxidant test component rather than the biological sample. According to this method, the antioxidant potential of the antioxidant test component against the oxidation reaction proceeding even in the presence of in vivo antioxidant components can be evaluated. The biological sample, the oxidizable substrates contained in the biological sample, the antioxidant components or materials that may be inherently contained in or externally added to the biological sample, the means for initiating and then continuing an oxidation reaction, and the quantification of oxidation products formed from the oxidizable substrates and the like are as described above. However, the biological sample is preferably invariable in its composition and antioxidant potential because the biological sample is merely one of test materials. Typically, a standard biological sample is prepared in advance. During evaluation, the biological sample may be mixed with the antioxidant test component or may be used as a control for comparison with the antioxidant test component, or its antioxidant potential may be used as a fixed value. Examples of the antioxidant test component include synthesized or naturally-occurring compounds which are expected to have antioxidant properties. These cannot be identified in their structures and sources in advance because they are compounds expected for their antioxidant properties in future. However, synthesized compounds include, for example, 4,6-di-t-butyl-5-hydroxy-2,2-di-n-pentyl-2,3-dihydrobenzofuran (hereinafter referred to as BO-653). The molar concentration ratio of the antioxidant test component to antioxidant components contained in the biological sample is 0.001-1000:1, preferably 0.002-500:1, more preferably 0.01-100:1, still more preferably 0.1-10:1.

### EXAMPLES

### Example 1: Assay reproducibility of oxidation products of cholesterol esters in plasma samples

Assay reproducibility was tested for oxidation products of cholesterol esters contained in plasma samples obtained from a normal human subject. In this test, the cholesterol esters were regarded as oxidizable substrates.

Blood was collected in heparin from one normal human subject who gave informed consent to prepare a plasma pool.

1.1 mL portions of this plasma sample were put into nine test tubes and were frozen-stored at -80°C.

Three test tubes A, B and C containing the frozen plasma were warmed to 37°C for 5 minutes to thaw the frozen plasma. After thawing, the test tubes were kept in ice.

10 µL of an aqueous solution of AAPH (available from Wako Pure Chemical Industries, Ltd.; Lot No. DWF 1183) was added to 300 µL of plasma removed from each test tube at an AAPH concentration of 10 mM. Then, they were warmed to 37°C to initiate an oxidation reaction.

After maintaining at 37°C for 8 hours, the 3 vessels each containing the reaction solution were immersed in ice to stop the reaction. 1 mL of a methanol containing 0.02% acetic acid and 5 mL of hexane were added to 100 µL of each reaction solution to extract lipids. 4 mL of the hexane extract removed from each reaction solution was concentrated on Speed Vac (AES1010 available from Thermo Savant), and each concentrate was dissolved in 200 µL of isopropyl alcohol.

Lipids were eluted by HPLC using a C18 reverse-phase column under the following conditions. UV detection was performed at 234 nm.

### HPLC conditions:

Column: C18 reverse-phase column (250 x 4.6 mm, LC-18 available from Supelco);
Mobile phase: ethanol : methanol : isopropanol : water = 2950:900:150:15;
Flow rate: 1.0 mL/min;
Temperature: room temperature;
Detection: UV detector (234 nm, Agilent 1100 UV variable wavelength detector, G1314A);
External standard: cholesterol linoleate hydroperoxide.

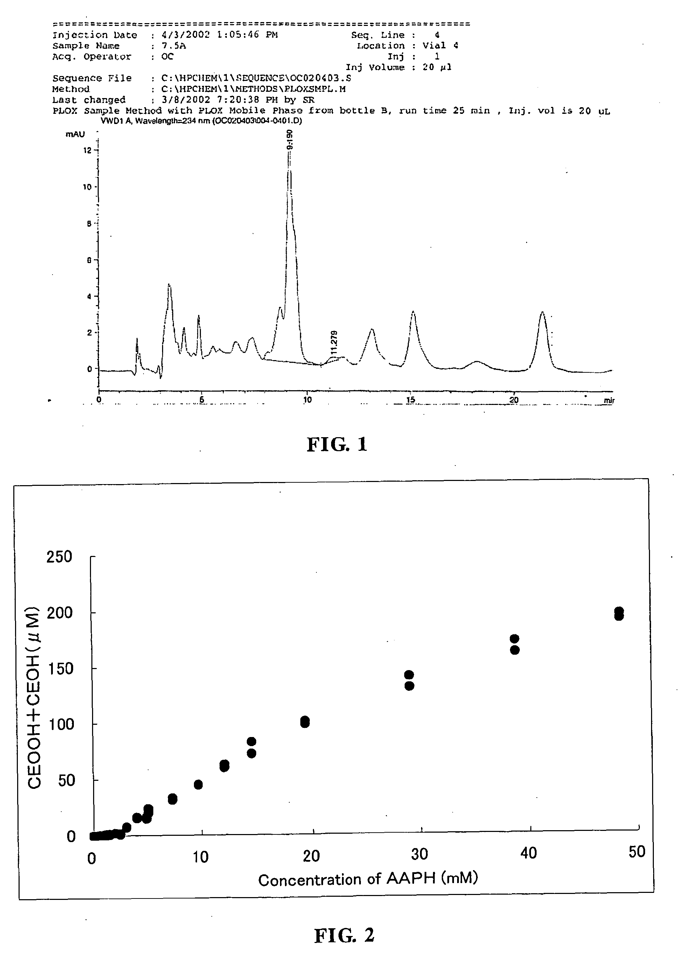

Under these HPLC conditions, oxidation products of cholesterol esters including both molecular species of cholesterol ester hydroperoxides (CEOOHs) and cholesterol ester hydroxides (CEOHs) were eluted at a retention time of about 9 minutes, and α-tocopherol was eluted at about 5 minutes. The presence of α-tocopherol shows that the oxidation reaction proceeded and then stopped in the presence of α-tocopherol in this test. FIG. 1 shows a representative HPLC chart in this assay.

The same assay procedures were performed on plasma samples contained in the remaining six test tubes. Among them, three tubes were tested on the day following the date of the assay described above (the 2nd day) and the remaining three tubes were tested two days after that date (the 3rd day). The results are shown in Table 1.

**Table 1: Assay reproducibility of CEOOHs and CEOHs**

| Assay date | Sample | CEOOH +CEOH (µM) | Intraday average ±S.D. (µM) | Intraday variation (CV%) | Interday average ±S.D. (µM) | Interday variation (CV%) |
|---|---|---|---|---|---|---|
| 1st day | A | 56.48 | 55.87±8.30 | 14.86 | 58.59±9.24 | 15.78 |
| | B | 47.28 | | | | |
| | C | 63.85 | | | | |
| 2nd day | A | 52.68 | 51.02±9.76 | 19.13 | | |
| | B | 59.84 | | | | |
| | C | 40.53 | | | | |
| 3rd day | A | 73.09 | 68.89±6.03 | 8.75 | | |
| | B | 61.98 | | | | |
| | C | 71.60 | | | | |

In Table 1, the "intraday variation" means the percentage of the standard deviation to the intraday average of samples A, B and C which were assayed on the same day, while the "interday variation" means the percentage of the standard deviation to the interday average on the 1st to 3rd days.

Table 1 shows that both intraday and internal variations (CV%) are within 20%. The overall variation among 9 samples is 18.2%. The variation in this range shows that oxidation products of cholesterol esters can be assayed with good reproducibility.

### Example 2: Dependence of the sustained presence of an antioxidant component on the concentration of AAPH

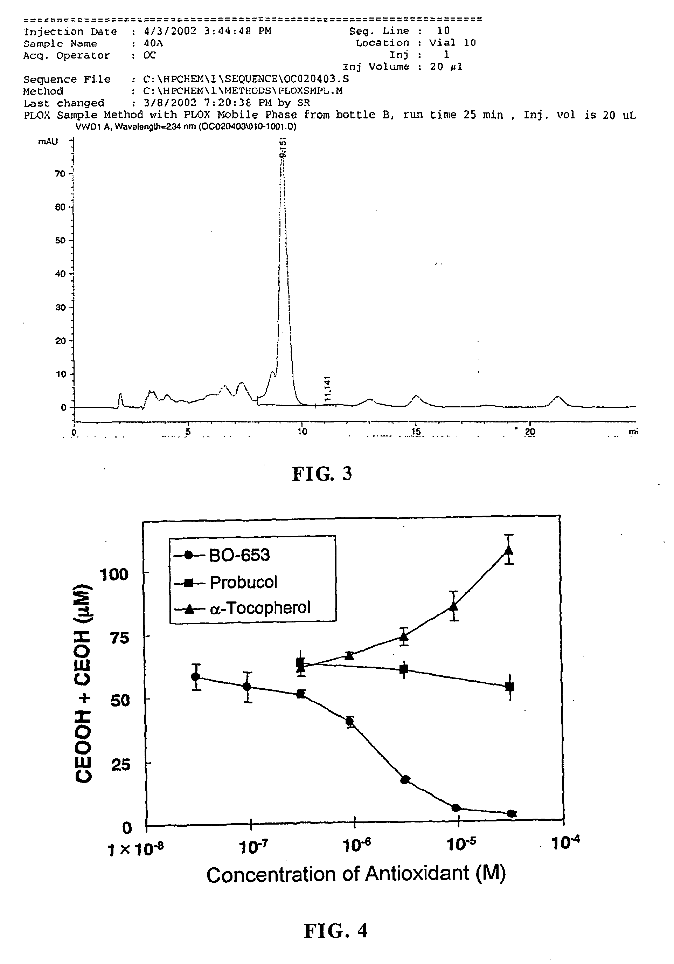

The dependence of the sustained presence of an antioxidant component on the AAPH concentration in assays of the present invention was examined by performing the same assay as in Example 1 except that the concentration of AAPH in plasma samples was varied from 0 to 48 mM. The results are shown in FIG. 2.

FIG. 2 shows that oxidation to CEOOHs and CEOHs proceeds at AAPH concentrations of 5 mM or more. However, HPLC showed that antioxidant component, α-tocopherol, disappears at AAPH concentrations of 29 mM or more. A representative HPLC chart of the oxidation reaction solution in which α-tocopherol has disappeared is shown in FIG. 3.

Thus, it was shown that when AAPH is used as an oxidation initiator, it should be added at a concentration of about 5-30 mM in order that the oxidation reaction of cholesterol esters may proceed in the presence of α-tocopherol.

### Example 3: Verification of the lipid oxidation-promoting effect of α-tocopherol

The influence of the type of antioxidant present in samples on the amounts of CEOOHs and CEOHs produced was examined by performing the same assay as in Example 1 except that antioxidant, α-tocopherol, probucol or 4,6-dit-butyl-5-hydroxy-2,2-di-n-pentyl-2,3-dihydrobenzofuran (BO-653), was added to plasma samples at various concentrations five minutes before AAPH was added. BO-653 can be prepared by the process described in WO94/08930, US 5,574,178 and EP 0665208A.

It has been reported that the rates of the reaction of probucol (available from SIGMA, Lot No. 61K1121) and BO-653 (available from Chugai Pharmaceutical Co., Ltd., Lot No. 009002) with linoleic acid peroxy radicals are 1/17.5 and 1/2, respectively, expressed as ratios to the rate of the reaction of α-tocopherol (available from ACROS Organics, Lot No. A015821301) with linoleic acid peroxy radicals (Gotoh N, Shimizu K, Komuro E, Tsuchiya J, Noguchi N, Niki E. Antioxidant activities of probucol against lipid peroxidations. Biochim Biophys Acta. 1992 1128:147-54; Noguchi N, Iwaki Y, Takahashi M, Komuro E, Kato Y, Tamura K, Cynshi O, Kodama T, Niki E. 2,3-Dihydro-5-hydroxy-2,2-dipentyl-4,6-di-tert-butylbenzofuran: design and evaluation as a novel radical-scavenging antioxidant against lipid peroxidation. Arch Biochem Biophys. 1997 342:236-43.). The determination of the rate of the reaction of a material with linoleic acid peroxy radicals is a conventional method for evaluating the antioxidant potential of the material. All of α-tocopherol, probucol and BO-653 have been evaluated by this conventional method to have an antioxidant potential.

The results are shown in FIG. 4. The concentrations of the antioxidants shown in FIG. 4 represent the values for only those externally added to plasma samples. In reality, endogenous α-tocopherol exists in all of the plasma samples. Thus, the concentrations of α-tocopherol in the samples to which α-tocopherol was externally added are higher than those indicated.

FIG. 4 shows that the amounts of CEOOHs and CEOHs produced significantly increase with the increase of the concentration of α-tocopherol while the amounts of CEOOHs and CEOHs produced significantly decrease with the increase of the concentration of BO-653. This indicates that α-tocopherol rather promotes oxidation of lipids such as cholesterol esters though it has been evaluated by the conventional evaluation method to have an antioxidant potential higher than BO0653

These results show that the conventional evaluation methods, which regards the total amount of in vivo antioxidant components contained in biological samples of which α-tocopherol is representative as the antioxidant potential of the sample, have not shown the true risk of lesions to be exacerbated by oxidative stress, and that the assay of the present invention can precisely evaluate such a risk since it is capable of detecting oxidation occurring even in the presence of in vivo antioxidant components.

### INDUSTRIAL APPLICABILITY

According to the present invention, in vivo antioxidant potential against oxidation reactions occurring in the presence of an antioxidant component such as α-tocopherol can be evaluated. Especially, the present invention is useful for clinical tests for conditions to be exacerbated by oxidative stress because oxidation reactions occurring in the presence of an antioxidant component α-tocopherol is considered to be critical for these conditions.

## Claims

1. A method for evaluating the antioxidant potential of a biological sample comprising the steps of:
(a) collecting a sample containing at least oxidizable substrates from a living body;
(b) initiating an oxidation reaction of said oxidizable substrates;
(c) continuing said oxidation reaction; and
(d) quantifying oxidation products formed from said oxidizable substrates by determining the rate of said oxidation reaction during the progress thereof or by performing an assay after said oxidation reaction has been stopped,
wherein said steps (b) and (c) are performed in the presence of one or more antioxidant components.

2. The method of claim 1 wherein said biological sample is blood, plasma or serum.

3. The method of claim 1 wherein one of said antioxidant components is α-tocopherol.

4. The method of claim 1 wherein said oxidation reaction is initiated with an oxidation initiator.

5. The method of claim 4 wherein said oxidation reaction is controlled by the concentration of said oxidation initiator at the start of said oxidation reaction so that said antioxidant components can continue to exist throughout said oxidation reaction.

6. The method of claim 4 wherein said oxidation initiator is 2,2'-azobis (2-amidinopropane) hydrochloride.

7. The method of claim 5 wherein said oxidation initiator is 2,2'-azobis (2-amidinopropane) hydrochloride and said concentration is 5-30 mM.

8. The method of claim 1 wherein said oxidizable substrates are lipids and oxidation products formed from said lipids include lipid oxides and lipid peroxides.

9. The method of claim 8 wherein said lipids are cholesterol esters, said lipid oxides are cholesterol ester hydroxides and said lipid peroxides are cholesterol ester hydroperoxides.

10. The method of claim 1 wherein said quantification of oxidation products formed from said oxidizable substrates is performed after stopping said oxidation reaction.

11. The method of claim 1 wherein said quantification of oxidation products formed from said oxidizable substrates comprises HPLC elution of analytes which allows said quantification.

12. The method of claim 11 wherein said analytes are detected with UV rays.

13. The method of claim 1, further comprising the step of adding an antioxidant material to said biological sample before performing said step (b) in the case where said biological sample does not contain a sufficient amount of antioxidant components to accomplish said steps (b) and (c) in the presence of the antioxidant components.

14. The method of claim 13 wherein said antioxidant material added to said biological sample is the same as one of antioxidant components inherently contained in said biological sample.

15. A method for diagnosing a disease or evaluating the prognosis and/or predicting the progress of said disease of a patient of interest on the basis of the antioxidant potential of a biological sample evaluated by the method of claim 1.

16. The method of claim 15 wherein said disease is a chronic endocrine disease, brain disease, pulmonary disease, hepatic disease, renal disease, circulatory disease, chronic inflammatory disease or cancer.

17. A method for evaluating the antioxidant potential of an antioxidant test component comprising the steps of:
(a) collecting a sample containing at least oxidizable substrates from a living body;
(b) adding said antioxidant test component to said biological sample;
(c) initiating an oxidation reaction of the mixture of step (b);
(d) continuing said oxidation reaction; and
(e) quantifying oxidation products formed from said oxidizable substrates by determining the rate of said oxidation reaction during the progress thereof or by performing an assay after said oxidation reaction has been stopped,
wherein said steps (c) and (d) are performed in the presence of one or more antioxidant components.

18. The method of claim 17, further comprising the step of adding an antioxidant material before performing said step (c) in the case where said biological sample does not contain a sufficient amount of antioxidant components to accomplish said steps (c) and (d) in the presence of the antioxidant components.
